# EUROPEAN PATENT SPECIFICATION

(11) **EP 4 373 555 B1**
(45) Date of publication and mention of the grant of the patent: **30.09.2026**
(21) Application number: 22754963.1
(22) Date of filing: 28.06.2022
(51) Int. Cl.: A61M 25/01, A61L 29/16, A61L 29/18, A61L 29/04, A61M 25/00

(54) **BIO-HYBRID MEDICAL DEVICE, SYSTEM FOR ADMINISTERING THERAPIES USING SUCH DEVICE**
BIOHYBRIDE MEDIZINISCHE VORRICHTUNG, SYSTEM ZUR THERAPIEVERABREICHUNG MIT EINER SOLCHEN VORRICHTUNG
DISPOSITIF MÉDICAL BIOHYBRIDE, SYSTÈME D'ADMINISTRATION DE THÉRAPIES METTANT EN OEUVRE UN TEL DISPOSITIF

(30) Priority: 23.07.2021 IT 202100019703
(43) Date of publication of application: 29.05.2024
(73) Proprietor: Scuola Superiore di Studi Universitari e di Perfezionamento Sant'Anna, 56127 Pisa (IT)
(72) Inventor: RICOTTI, Leonardo, 56127 PISA (PI) (IT); IBERITE, Fedreica, 56127 PISA (PI) (IT); VANNOZZI, Lorenzo, 56127 PISA (PI) (IT)
(74) Representative: Tiburzi, Andrea
(86) International application number: PCT/IT2022/050185
(87) International publication number: WO 2023/002514

(56) References cited:
- EP-A1- 1 800 824
- US-A1- 2008 021 416
- RAMAN RITU ET AL: "Optogenetic skeletal muscle-powered adaptive biological machines", vol. 113, no. 13, 29 March 2016 (2016-03-29), pages 3497 - 3502, XP055910841, ISSN: 0027-8424, Retrieved from the Internet <URL:http://dx.doi.org/10.1073/pnas.1516139113> [retrieved on 20220408], DOI: 10.1073/pnas.1516139113
- GUIX MARIA ET AL: "Biohybrid soft robots with self-stimulating skeletons", vol. 6, no. 53, 28 April 2021 (2021-04-28), pages 1 - 13, XP055910843, Retrieved from the Internet <URL:http://dx.doi.org/10.1126/scirobotics.abe7577> [retrieved on 20220408], DOI: 10.1126/scirobotics.abe7577

## Description

The present invention relates to a bio-hybrid medical device, system for administering therapies using such device, and method (not claimed) of orientation in space thereof.

### Field of the invention

More in detail, the invention relates to a medical device, such as a catheter or microcatheter, designed and manufactured in particular to allow the administration of drugs in very small districts, but which can be used for any case in which it is necessary to reach extremely narrow areas of the body to deposit substances or perform operations.

In the following, the description will be addressed to a microcatheter for drug administration, but it is clear that it should not be considered limited to this specific use.

### Prior art

As is well known, many clinical treatments are based on the use of systemic pharmacological therapies, which involve the injection or administration of drugs, which enter the bloodstream and are transported to all parts of the body, including "target" tissues to be treated locally.

However, often the absorption of a drug, if excessively diffused into the bloodstream, involves a considerable number of side effects, caused by the intrinsic toxicity of the drug itself, combined with a non-specific effect at the level of the organs to which the treatment would not be aimed to (see [1], [2]).

In recent decades, the solutions to this problem have shifted from the use of drugs with a non-selective mechanism of action, to targeted therapy with drugs or nanometric vectors functionalized towards a specific molecular target. These drugs or vectors generally have as their objective a particular target, characterizing the diseased phenotype (see [2]).

For example, in some tumors, the effect of increased permeability and retention of capillaries near primary tumors is exploited for the passive accumulation of the drug *in situ,* and consequent selective identification of diseased cells, thanks to the recognition of specific membrane receptors, or with agents responsive to pH, temperature, or other endogenous physical stimuli.

The "targeted therapy", that is the therapy that involves the administration of drugs "in situ" is considered to have a very promising potential in the treatment of various pathologies, including solid tumors, normally treated with chemotherapeutic agents, characterized by high cytotoxicity.

A problem still felt in the sector is the fact that for this type of therapy the drug must be administered and accumulated as close as possible to the specific site of action or target area, limiting its so-called non-specific distribution.

In the case of solid tumors, selective targeting is allowed by the use of engineered nanoparticles (see [3]). In current nanomedicine, it is estimated that only 0.7% of the administered nanoparticles normally reach a specific site, for example, related to a solid tumor (see [4]). The distribution in the systemic circulation of nanomaterials can in fact involve their capture by the reticuloendothelial system, whose role is to eliminate toxic and "extraneous" substances from the bloodstream (see [5]).

Despite the nano-bio-technological advancement in the sector, this biological process involves the accumulation of nanomaterials in organs such as the spleen and kidneys with consequent toxic effects (see [6]). This also implies that, in order to get a sufficient number of nanocarriers to the target area to be treated, it is necessary to inject a high concentration of these vectors. Therefore, the therapies are effective only when high doses of the therapeutic agent are administered to overcome the aforementioned problem and allow the desired effect to be achieved.

To overcome this problem, the use of intravascular microcatheters, i.e. flexible miniaturized devices, is widespread for the treatment of numerous pathologies, which allow approaching the site of interest by infusing the therapeutic agent in the vicinity of the target site, thus favoring an accumulation of drug to the target and limiting its spread to other healthy tissues (see [7] - [9]). However, the catheters currently available on the market are not optimal for this purpose.

In fact, in a typical procedure with vascular access, a catheter (or microcatheter; in the following, we will use the term "catheter" meaning indifferently "microcatheter"), or a similar tubular medical device, is inserted through the skin, inside a vein at the level of the patient's neck, arm or leg, to reach the specific site to be treated through a more or less "tortuous" network of arterioles and capillaries.

As is known, a catheter normally consists of a "proximal" termination, close to the operator, and a "distal" termination, which can be inserted into the patient's body and, therefore, far from the operator who maneuvers the catheter.

Once manual insertion of the distal termination by the operator has taken place, the catheter, i.e. the distal termination, must be guided or conducted. This conducting maneuver is performed with distal maneuvers on the catheter, performed by the operator by acting on the proximal end of the device. In addition, the use of imaging techniques is envisaged, to orient said distal termination in the patient's body, such as fluoroscopy based on the use of X-rays, real-time magnetic resonance imaging (see [10], [11]), and ultrasound (see [12]).

The problems related to this type of operation are mainly due to the difficulty of making the distal termination perform an orientation in three dimensions, through a proximal manipulation of the device, performed outside the patient (by hand or with a robotic platform), under the guidance of real-time images.

The effectiveness of the operation is strongly dependent on the coordination capacity and on the experience of the operator himself. Furthermore, the possibility of rotating or twisting the distal termination by proximal actions is more limited as the length of the inserted section, the "tortuosity" of the section itself, and the number of curves already made by the catheter increase.

It is known to use devices, which use magnets positioned externally to the patient, to orient the related catheters or devices. This solution has been one of the main and most used solutions to guide said devices inside small blood vessels (see [13]).

However, this type of orientation does not always allow effective guidance of the device to be guided (e.g., a catheter or the like), especially through tortuous paths. Various control systems have been proposed in the field to improve this solution. For example, it is known the introduction in the distal end of the catheter, of elements such as small ferromagnetic spheres, micro-coils or permanent magnets, to facilitate their orientation, although many disadvantages are also known, which limit their use (see for example [14 ]).

The application of ferromagnetic spheres can, for example, introduce unwanted "artifacts", due to dipole-dipole interactions, and cause possible friction during the actuation of the tip on the internal walls of the vessels.

In the case of the application of micro-coils, the manufacture of the latter requires the use of very sophisticated techniques, which entail their limited availability due to their small size. Furthermore, resonance phenomena of the coils themselves can occur, for which generally difficult to integrate heat reduction systems are needed, since the use of electromagnets can generate excessive and unwanted heat. Furthermore, said micro-coils require relatively high magnetic fields to allow the control of the distal part of the catheter or the relative device.

Finally, the use of permanent magnets as an actuation means does not allow, with the current technology, obtaining an efficient control in guiding the catheter without the physical support given by the contact with the blood vessels.

The main limitation of magnetic navigation is the difficulty of applying magnetic fields in the working area, to actuate different areas of the catheter in the event of the presence of multiple permanent magnets, since the magnetic field necessarily affects the entire working area (see [15 ]).

Furthermore, many actuation systems employ mathematical models and algorithms relating to catheters equipped with a single magnet at the tip, which, however, have more limited performance than a multiple use of magnets.

The magnetic guide can, therefore, help get closer to the target, albeit with some restrictions. The position and orientation of the tips can be controlled using the magnets in the catheter and an appropriate external magnetic navigation system. However, this criterion still has further limitations if the goal is an actuation at the tip (the distal part) of the catheter.

Furthermore, catheters guided with axially magnetized magnets cannot generate the twisting motion necessary to facilitate the tip direction (see [16]). A magnetic catheter with a diametrically magnetized magnet can generate a rotation/perforation movement, although this configuration could cause structural problems, due to the torsion between the magnet and the catheter, and confers a limited dragging capacity (i.e. *steering*) (see [17]).

Furthermore, it is also necessary to mention the possible scalability problems of the magnetic coupling, which would make it difficult to manage the control of the magnetic catheter in deep areas and tissues, especially in small districts such as arterioles, which would require very small catheter dimensions (see [13]).

Furthermore, it should be considered that the currently available level of miniaturization of the control systems of the distal part of the catheter or medical device, generally allows for limited manipulation of the catheters, forcing operators to release the therapeutic agent more "upstream" than the area or area of the body to be effectively treated.

This has the consequence of having a less effective action (with the same drug released) and undesirable effects on healthy tissues, due to the "dispersion" of the drug itself in the branches of the vascular network that precedes said area to be treated. In particular, considering the current construction scales, it is not possible to build miniaturizable artificial motors down to a few tens of microns, capable of exerting appreciable forces and torques (see [18]).

It should also be considered that for this specific medical application, any motors used should be perfectly biocompatible and haemo-compatible. This technical result is technologically very complicated to achieve with the currently available technologies.

It appears evident that the lack of accurate distal actuation of the tip of a catheter or medical device generally limits the degrees of freedom of the same, not allowing a suitable control to orientate itself in a capillary vascular system, effectively reaching the target areas, up to the level of the small arterioles or even the capillaries inside the body.
Documents D1 US2008/021416 A1; EP1800824 A1; Raman Rutu, et al, "Optogenetic skeletal muscle-powered adaptive biological machines", Proceedings of the National Academy of Sciences; and Guix Maria, et al, "Biohybrid soft robots with self-stimulating skeletons", Science Robotics are representative of the available art.

### Scope of the invention

In light of the above, it is, therefore, the object of the present invention to propose a medical device that allows the maximization of the effectiveness of a targeted therapy, along the tortuous path of arteries and arterioles that separates the entry point of the catheter itself from the target tissue or area, such that, once the latter have been reached, it is possible to release therapeutic agents or the like.

It is also an object of the present invention to propose an intravascular microcatheter, or a medical device in general, which allows administering therapeutic agents in proximity to targets inside the body, allowing the execution of the movement in various directions.

A further object of the present invention is to provide the tools necessary for carrying out the method and the apparatuses that carry out this method.

### Object of the invention

These and other results are obtained according to the invention with a hollow composite magnetic field responsive microcatheter with a tip having a variable diameter in the range of 50-500 µm, which allows reaching areas of the body impossible to reach with traditional technologies and release therapeutic agents in these areas or perform other operations.

The microcatheter is made of a soft, biocompatible and deformable material.

The outer wall also has a blood-compatible coating to make the microcatheter usable for intravascular applications.

The device is miniaturized and can be dragged by magnetic fields, which allow the operator to control the movements of the catheter from the outside in a controlled and non-invasive way.

The microcatheter is made using muscle cell-based actuators that can scale down to micrometer size without losing their efficiency. Muscle cells have micrometric dimensions, are naturally biocompatible, can grow, adapt, and self-repair, and have the ability to self-assemble even in vitro in the formation of functional and contractile tissues.

The contraction of skeletal muscle tissue can be controlled by external stimuli. Cells are able to convert chemical energy into mechanical work, not requiring bulky energy sources (such as batteries and the like) but only glucose and protein factors that are present in the blood itself.

In particular, the execution of the movement in various directions is allowed by the presence on board of biohybrid actuators based on induced pluripotent stem cells (iPSC - Induced Pluripotent Stem Cell), deriving from the patient himself (which therefore are not subject to the risk of rejection or other immune reactions), possibly optogenetically modified, so as to guarantee their activation and contraction by means of luminous stimuli sent proximally by the operator.

Therefore, it is specific object of the present invention a medical device for releasing drugs, functionalized nanometric vectors, medical equipment, and the like, as defined in claim 1, comprising a catheter, having one proximal end and one distal end, which, in use, can be inserted into the blood vessels or a body area of a patient, wherein said distal end is configured for releasing said drugs, functionalized nanometric vectors, medical equipment and the like, characterized in that it comprises one or more bio-hybrid actuators, arranged in correspondence of said distal end of said catheter, wherein said one or more bio-hybrid actuator can be actuated by means of actuation signals, for orienting said distal end of said catheter.

Always according to the invention, said device may comprise four distally integrated bio-hybrid actuators.

Still according to the invention, said one or more bio-hybrid actuators may be based on cells differentiated starting from iPSC (Induced Pluripotent Stem Cell), preferably derived from the patient himself.

Advantageously according to the invention, said one or more bio-hybrid actuators may be based on optogenetically modified cells, said actuation signals may be electromagnetic radiations, in particular optical control or actuation signals, for actuating said one or more bio-hybrid actuators, and said catheter may have an actuation portion, arranged in correspondence of said at least one bio-hybrid actuator, through which said control electromagnetic radiations pass for reaching said at least one bio-hybrid actuator for actuating it.

Further according to the invention, said catheter may have a longitudinal channel, and said catheter may comprise inside said longitudinal channel an optical fiber, for transmitting said actuation optical signals.

Always according to the invention, said longitudinal channel may have an internal reflecting wall, for reflecting and transmitting along the catheter said actuation signals.

Still according to the invention, said internal reflecting wall that carries out said optical fiber may be coated with a layer composed of gold nanoparticles.

Advantageously according to the invention, said internal reflecting wall that carries out said optical fiber may be coated with alginate treated with calcium chloride, for providing a multilayer coating for the propagation of control electromagnetic radiation in said longitudinal channel.

Further according to the invention, said catheter may comprise an optical fiber having a cylindrical shape, so as to identify said longitudinal channel.

Preferably according to the invention, said actuation portion may be an opening, for allowing the passage of said control electromagnetic radiations of said actuation signal, or it is a portion of said transparent catheter, so as to allow the transmission of said control electromagnetic radiations of said actuation signal for reaching said one or more bio-hybrid actuators.

Always according to the invention, said one or more bio-hybrid actuators may be each made of different groups of cells, optogenetically modified so as to respond to light radiation at different wavelengths, so that each of said bio-hybrid actuator can be actuated independently when control electromagnetic radiations are transmitted through said longitudinal channel.

Still according to the invention, each of said bio-hybrid actuators may comprise a couple of electrodes, said device may comprise electric tracks associated with each bio-hybrid actuator, so that a respective bio-hybrid actuator can be selectively actuated by means of electrical actuation signals.

Advantageously according to the invention, said electric tracks may be integrated in said catheter or inserted in said longitudinal channel.

Further according to the invention, said catheter may comprise particles responsive to magnetic fields incorporated in the material of which it is made, as micro/nanoparticles of iron oxide (Fe3O4), neodymium iron boron (NdFeB) or maghemite (*γ*-Fe2O3).

Preferably according to the invention, said catheter may be made of biocompatible elastomeric materials, such as polydimethylsiloxane (PDMS) and/or polyurethanes that can be polymerized by temperature, electromagnetic or condensation radiation, wherein said polyurethanes are modulated in stiffness/elasticity as a function of isocyanate and hydroxyl groups, and/or it comprises synthetic immuno- and emo-compatible hydrogels that can be modulated in stiffness, such as polyethylene glycol (PEG), in which the stiffness, comprising diacrylate groups (PEGDA).

It is further object of the present invention a system for administering therapies for releasing drugs, functionalized nanometric vectors, medical equipment and the like, comprising a medical device as described above, wherein said distal end of said catheter can be inserted into the blood vessels or the body of a patient, a control unit connected to the proximal end of said catheter, for introducing signals with appropriate light radiation, to be transmitted by means of said longitudinal channel up to said bio-hybrid actuators, for selectively activating them, and a navigation unit capable of interacting by means of generating a magnetic field with the particles incorporated in the material of which the catheter is made, so as to be able to move said catheter.

Also disclosed but not part of the claimed invention and left for illustrative purposes is a method for controlling a system for administering therapies according to the preceding claim, which comprises the step of introducing a light signal at a predefined wavelength in said longitudinal channel for selecting and actuating said one or more bio-hybrid actuators on the basis of the wavelength of control electromagnetic radiation inserted in said longitudinal channel. This also applies to the methods for controlling described further below.

### Brief description of the figures

The present invention will be now described, for illustrative but not limitative purposes, according to its preferred embodiments, with particular reference to the figures of the enclosed drawings, wherein:
figure 1 shows an overall perspective view of a medical device according to the present invention;
figure 2 shows a longitudinal sectional view of a variant of a catheter of the medical device according to the invention;
figure 3 shows a side view of a second embodiment of a medical device according to the present invention;
figure 4 shows a detail of the longitudinal channel of the catheter of the medical device according to figure 3;
figure 5 shows a further detail of the catheter of the medical device according to figure 1;
figure 6 schematically shows a first manufacturing process of the catheter of the medical device according to the present invention;
figure 7 schematically shows a second manufacturing process of the catheter of the medical device according to the present invention;
figure 8 shows the orientation of the medical device according to the present invention in a blood vessel;
figure 9 shows four different orientations of the medical device according to the present invention;
figure 10A shows a first method of integrating the actuator means to the catheter of the device according to the present invention;
figure 10B shows a second method of integrating the actuator means to the catheter of the device according to the present invention;
figure 10C shows a third method of integrating the actuator means to the catheter of the device according to the present invention;
figure 10D shows a fourth method of integrating the actuator means to the catheter of the device according to the present invention;
figure 11 shows six stages of entrainment and orientation of the medical device inside capillaries;
figure 12A shows the achievement by means of the catheter according to the invention of a target body region;
figure 12B shows the achievement by means of a catheter according to the prior art of a target body region; and
figure 13 shows a second embodiment of a medical device according to the present invention.

### Detailed description

In the various figures similar parts will be indicated with the same numerical references.

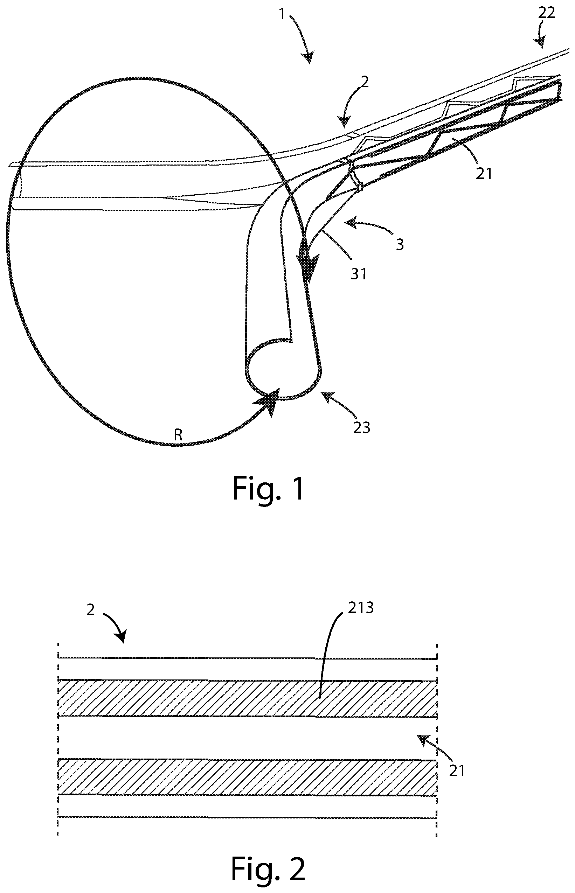

With reference to figure 1, a first embodiment of a medical device 1 according to the present invention is observed, essentially comprising a catheter 2 and actuator means 3.

The catheter 2 is internally and longitudinally hollow, presenting a longitudinal channel 21.

Furthermore, said catheter 2 comprises a proximal end 22, which, in use, is close to the operator, and a distal end 23, which, in use, can be inserted into the patient's body and, therefore, far from the operator operating the medical device 1.

In a first variant, the catheter 2 has an internal reflecting wall 211, intended to reflect light radiation, such that said longitudinal channel 21 acts as an optical fiber for the transmission of optical type actuation signals, at different wavelengths, as will be better explained below.

In a further variant, the catheter 2 can comprise an optical fiber 213, having a cylindrical and hollow shape, when viewed in cross-section, so as to identify said longitudinal channel (21). In this way, the longitudinal channel 21 can be functional or allow the introduction of operating members, for example, means or devices for the injection of drugs, functionalized nanometric vectors, medical equipment, and the like. In this case, the optical radiation that can be transmitted through the optical fiber 213, as will be more fully explained below, can only propagate through it, without passing through the volume of the longitudinal channel 21 of the catheter 2.

At the distal end 23, the catheter 2 has an actuation portion 212, which allows the light radiation in correspondence of the actuator means 3, the function of which will be better explained below.

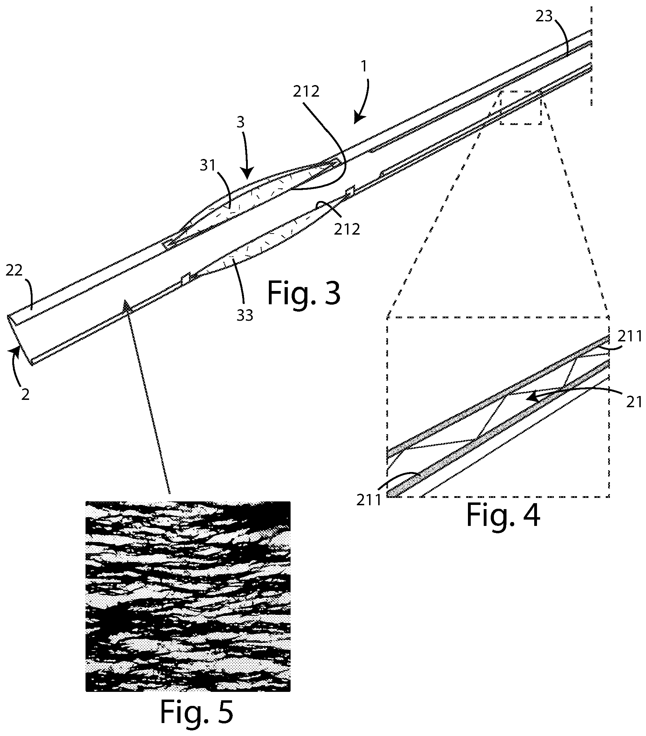

Referring also to figures 3 and 4, a second embodiment of the medical device 1 according to the present invention is also observed, which differs from that of figure 1 in that the actuator means 3 provide a plurality of distally integrated biohybrid actuators, as will be better described below. Therefore, for convenience, the two embodiments will be described together, specifying in the following any functional and structural differences.

In one embodiment the catheter 2 of the medical device 1 (as shown in figure 1 or figure 3) is manufactured by the use of biocompatible elastomeric materials, for example, polydimethylsiloxane (PDMS), or polyurethanes that can be cured by temperature, by means of light (UV or visible) or by condensation.

Polyurethanes can be modulated in stiffness/elasticity, according to the isocyanate and hydroxyl groups that are made reacting, and have an optimal hemocompatibility, as demonstrated by their extensive use in the cardiovascular field.

In other embodiments, the use of immuno- and hemo-compatible synthetic hydrogels that can be modulated in stiffness is provided. An example of a hydrogel that can be modulated in stiffness is polyethylene glycol (PEG), which can also be photo-responsive in the form that includes diacrylate groups (PEGDA), and whose stiffness can be modulated, for example, by varying the formulation or the content of the photo-initiator.

Figure 5 schematically shows the catheter structure 2.

In other embodiments of the catheter 2 other modified versions of PEGDA can be used, such as those modified with polyacrylamide (see [21]).

The catheter 2 also comprises particles responsive to magnetic fields incorporated in the material, which it is made of. More particularly, in some embodiments, micro/nanoparticles of iron oxide (Fe₃O₄), neodymium iron boron (NdFeB) or maghemite (γ-Fe₂O₃) are included in the catheter 2.

In some embodiments, magnetic composite materials can be manufactures when the polymeric and magnetic components are mixed first.

This process is followed by a step of spatial orientation of the powders by applying a permanent magnet, in the phase in which the polymer is not yet completely solidified.

In the case of ferromagnetic powders (e.g., neodymium iron boron, NdFeB), the application of a fairly intense magnetic field, that is of the order (in a very indicative way) of 1-10 Tesla, allows a permanent magnetization of the powders themselves, in order to optimize both the necessary magnetic field and the necessary concentration of particles.

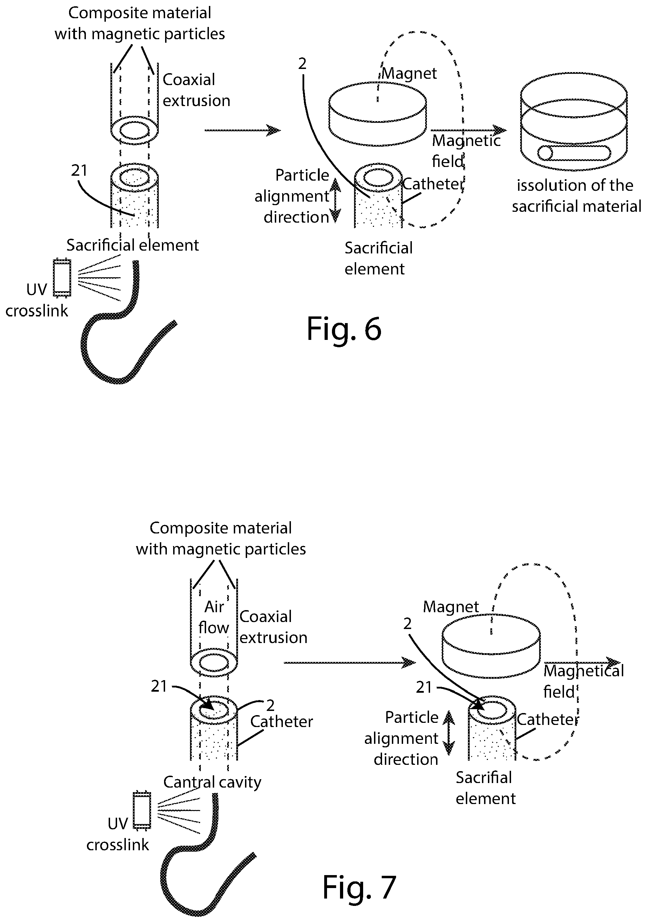

With reference to figures 6 and 7, it is possible to observe an example of the manufacturing process.

In particular, figure 6 schematically shows the manufacturing process of the hollow fibers and the alignment of the magnetic particles, in the presence of the internal sacrificial layer (figure 6), and in the absence of the internal sacrificial layer (figure 7).

As can be seen, the hollow polymeric fibers can be obtained by casting through the use of expendable and non-expendable molds, or by coaxial spinning/molding processes.

Spinning can be carried out from melt (so-called *melt spinning), dry* (so-called *dry spinning*)*,* or wet (so-called *wet spinning*)*.*

In other embodiments, the catheter 2 can be made by means of the further method represented by electrospinning, which exploits the electrostatic force generated by a source of high voltage potential to form an electrically charged jet, which is ejected from a capillary and gives rise to the fiber.

In all the above procedures known in the art, the longitudinal channel 21 can be created by means of the use of an internal sacrificial layer, which can be soluble in water (e.g., polyvinyl acid, as described in [22]), or other polymers soluble in solvents compatible with the manufacturing process of the catheter 2.

Alternatively, the longitudinal channel 21 can be made by applying an air flow (see figure 7).

To create the optical fiber inside the longitudinal channel 21 that allows the reflection of the internal light of the optical actuation signals up to the end or distal zone 23 of the catheter 2, the fiber manufactured with elastomers (e.g., PDMS), and in particular the internal reflective wall 211, can be covered with a layer composed of gold nanoparticles, which can be deposited in the activated surface, and can create an external layer by self-assembly with the intermediary of the positively charged molecule of polydialldimethylammonium chloride (PDDA) ( see [23]).

In the case of using hydrogels, the fiber can be further coated with alginate, followed by treatment with calcium chloride, which is repeated to provide a multilayer coating that ensures the propagation of light inside the longitudinal channel 21, forming the so-called cladding layer (see [24]).

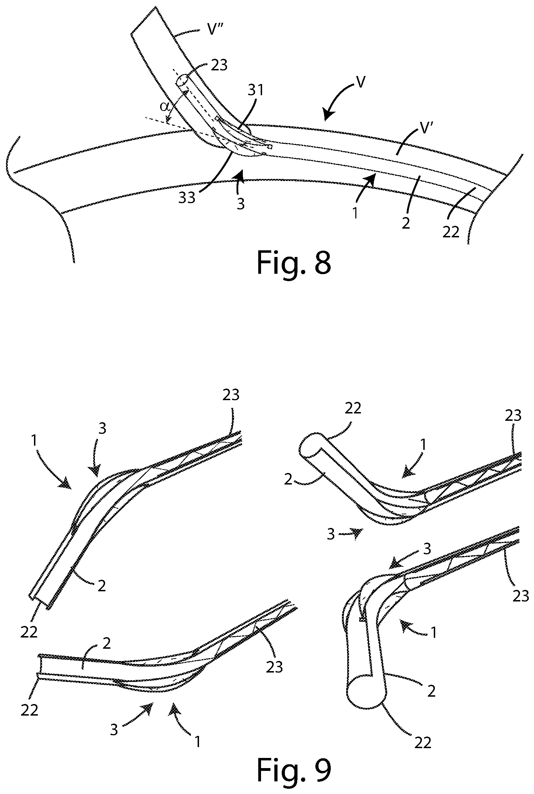

The actuator means 3 are intended to actuate the catheter 2 at the tip, and in particular at the distal end 12. Said actuator means 3 are arranged at the distal end of the medical device 1, so as to allow, with reference to figure 8, the orientation of the distal end 12 of the medical device 1, to allow its orientation within a medical district, and in particular in blood vessels and capillaries.

In the embodiment shown in figure 1, said actuator means 3 comprises a single distally integrated biohybrid actuator, indicated with the numeral reference 31.

In the embodiment shown in figure 3, said actuator means 3 comprises four distally integrated biohybrid actuators, indicated with the reference numbers 31, 32, 33, and 34.

The catheter 2 of a medical device 1 according to the embodiment of figure 3 is observed in said figure 8. In particular, it is observed how the activation of the actuator 31 of the actuator means 3, by contracting, allowed to orient the distal end 12 inserted in the portion V' of the vascular duct V' of the blood vessel V, towards the capillary tract V" of the same blood vessel V, bending the catheter 2 by an angle *α*, as shown in the figure. Since there are four biohybrid actuators 31, 32, 33, and 34, they allow an orientation of the distal end 12 of the medical device 1 in the space in the present embodiment, as will be better described below.

In one embodiment said biohybrid actuators 31, 32, 33, and 34 are based on cells differentiated starting from iPSC (Induced Pluripotent Stem Cell)), preferably derived from the patient himself. Single muscle cells can develop a force of contraction in the order of a few micronewtons (see [19]), reaching up to 5-10 mN in differentiated and mature tissues (see [20]).

Said actuation portion 212 (see in particular figure 3), which, as said, is positioned at the distal end 12 of the medical device 1, the one in which the biohybrid actuators 31, 32, 33, and 34 are arranged, the catheter 2 is uncovered, that is, it is transparent to the passage of the light towards the actuators, or rather of the control electromagnetic radiation, previously reflected internally in the longitudinal channel 21 up to that point, being said biohybrid actuators 31, 32, 33, and 34 activable.

In particular, said actuation portion 212 is realized as an opening, suitable to allow the passage of light, or, in other embodiments, in transparency, i.e., the catheter 2 is partially transparent, to allow the transmission of light and the achievement of the transmitted light radiation through the longitudinal channel 21 to the four biohybrid actuators 31, 32, 33, and 34.

Said four biohybrid actuators 31, 32, 33, and 34 are each made with different groups of cells, differentiated as each subjected to different optogenetic modifications (inducing the expression of different so *channelrhodopsins*)*,* in order to respond to light at different wavelengths, effectively enabling four different degrees of freedom of the medical device 1.

Among other things, said biohybrid actuators 31, 32, 33, and 34 can be activated independently, transmitting the specific activation light radiation as better defined below, or simultaneously, transmitting more light radiations with the same light signal.

Therefore, as can be seen in figure 9, light stimuli at different wavelengths allow the bending of the catheter 2 in different directions, allowing very precise control of the same. In particular, due to the four biohybrid actuators 31, 32, 33, and 34, it is possible to orient the distal end 23 of the catheter 2 of the medical device 1 according to four different orientations or directions, according to the relative arrangement of the biohybrid actuators 31, 32, 33, and 34. In the medical device 1 of figure 1, in which the actuator means comprises a single biohybrid actuator 31, the distal end 23 can be bent in one direction only. In this case, to orient the medical device 1 within a tortuous path, it will be necessary to rotate it according to the arrow indicated with R along the longitudinal axis of the catheter 2 itself, as shown in the same figure 1. Naturally, for very long catheters 2, it appears easier to orient a medical device 1 according to the second embodiment shown in figure 3, which provides for more degrees of freedom.

The use of iPSC for the realization of the biohybrid actuators 31, 32, 33, and 34 allows realizing a sort of "autologous" instrument, perfectly bio- and immunocompatible. Furthermore, the integration of these cells (whose function is based on biochemical transductions) allows the system to be maintained with the nutrients present in the patient's own blood, without the need to integrate batteries, thus also reducing the overall size.

Furthermore, specific protocols are known to differentiate different cell types starting from iPSCs. Different phenotypes can be kept in co-culture or separately grown. For example, cultures of cells differentiated exclusively into skeletal muscle or co-cultures of iPSCs differentiated into skeletal muscle and motor neurons can be used for the integration into the catheter 2.

More particularly, in embodiments, it is possible to provide a procedure based on the removal of somatic cells, such as skin fibroblasts, by providing suitable reprogramming factors, thus obtaining the generation of patient-specific iPSCs.

Subsequently, it is possible to carry out in vitro seeding and differentiation on micro-nano films, by subsequently carrying out a culture of muscle cells only, or a co-culture of muscle cells and motor neurons, so as to obtain the integration with the catheter 2.

Alternatively, in vitro seeding and differentiation can always be carried out on the device, subsequently carrying out the culture of only MS muscle cells, or the co-culture of muscle cells and motor neurons.

In particular, the cells are seeded and grown/differentiated either directly on the device, or on non-degradable elastomeric micro/nanofilms, and then assembled on the external walls of the catheter 2, as shown in figure 3.

These structures can be composed of a mixture of elastomers or hydrogels with an adjustable stiffness according to the force expected by the muscle cells, so that the catheter 2 can be deformed by cell contraction. Taking into account a contraction force of the single cell around 1 µN (see [19]) it is possible to consider a typical catheter with a soft tip, namely with an elastic modulus of the order of ~ 10 kPa - 1 Mpa, with an external diameter between the 50 and 500 µm, to produce a flexion between 20 and 100°, which allows high mobility and directionability of the distal end 12 of the medical device 1. Among other things, for reduced thicknesses, the suitable elastic modulus can be even higher than the interval indicated above, which, therefore, has to be considered entirely indicative and not limiting.

Some materials that can be used for the manufacture of micro/nanofilm or catheter tip 1 are PDMS, polyacrylamide, poly (styrenebutadiene), polylactic acid (PLLA), polystyrene (PS), and other polymers, possibly mixed together with nanomaterials (e.g., nanoclays, ceramic nanoparticles, etc.), to increase the manipulability and robustness of the micro/nanofilms, and the echogenicity (to enable the vision of the device by ultrasound), in the case of ceramic nanoparticles.

These systems must be kept stable until the end of iPSC differentiation, i.e., for about 7-10 days for the exclusive culture of differentiated iPSCs in skeletal muscle, or 14-20 days for the co-culture of skeletal muscle and motor neurons (see [25] and [26]), and for use in endovascular applications, i.e., within approximately 90 minutes for the treatment of solid tumors, for example.

The differentiation of iPSCs is made in vitro, and it is addressed, due to specific known differentiation protocols that require the use of factors and nutrients that promote differentiation towards a particular phenotype.

Furthermore, as regards the muscle phenotype, the alignment of the cells along the direction of contraction is an essential parameter for the optimal differentiation and contraction.

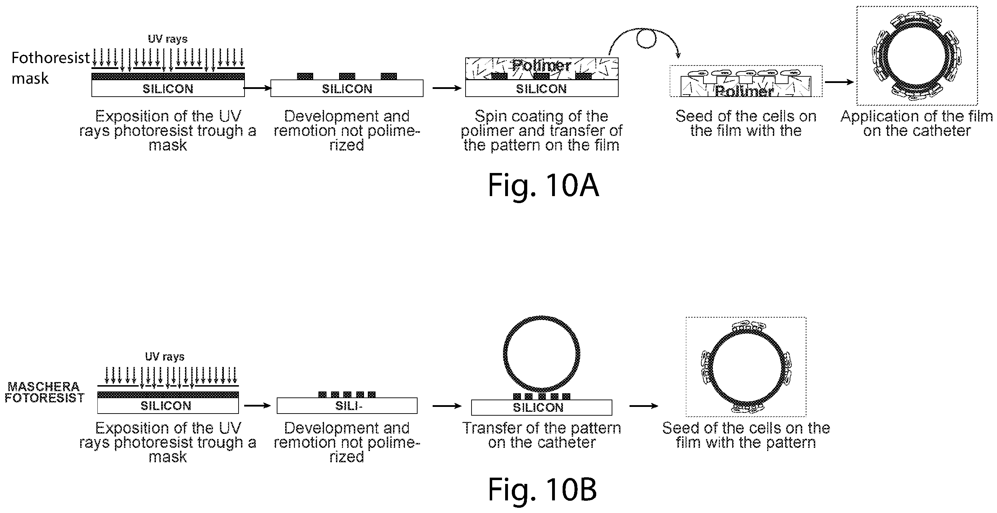

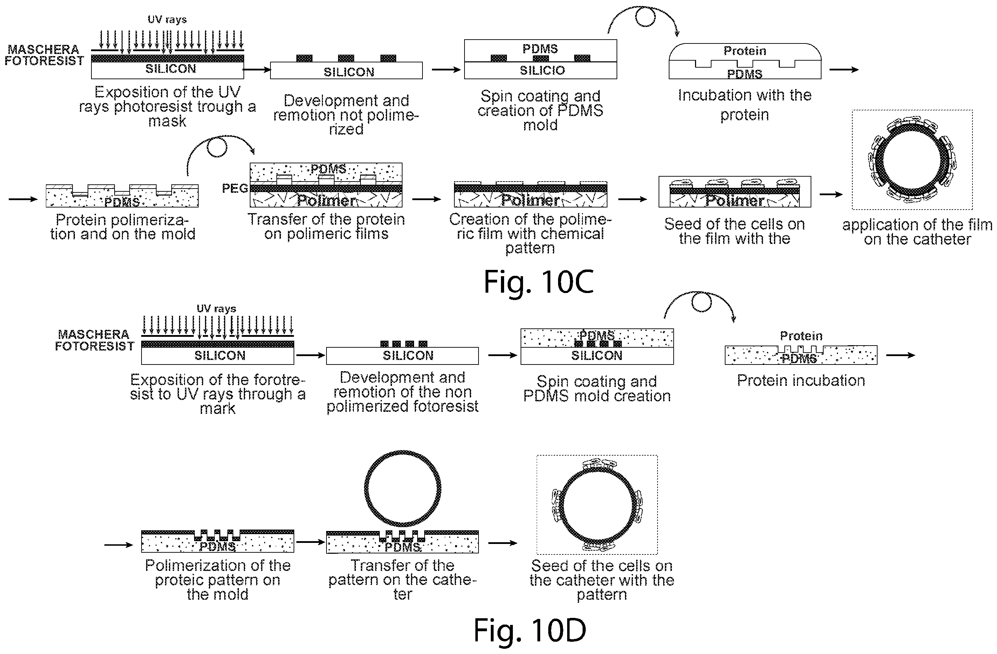

Referring to figures 10A, 10B, 10C, and 10D, examples of substrate manufacturing processes for the cell growth with anisotropic patterning and possible ways of integrating the biohybrid actuators 31, 32, 33, and 34 on the medical device 1 are observed.

The indicated methods are basically two:
A. fabrication of microgrooves by photolithography techniques (physical patterning) on the films to be applied on the catheter (see figure 10A) or directly on the catheter itself (see figure 10B)
B. creation of protein strips or adhesive and prodifferentiative protein matrices (e.g., Matrigel, vitronectin, collagen, poly-L-lysine), which alternate with non-adhesive substances such as polyethylene glycol (protein patterning). Similarly, protein patterning can be done on the films to be applied on the catheter (see figure 10C), or directly on the catheter itself (see figure 10D). The presence of such anisotropic stimuli is known to favor differentiation towards a muscle phenotype (see [27] and [28]).

More specifically, referring to figure 10A, we observe a physical patterning process on micro/nano polymeric films, subsequently applied on catheter 2.

In figure 10B a physical patterning process directly on catheter 2 is observed.

In figure 10C a protein patterning process on polymeric micro/nano films, subsequently applied on catheter 2, is observed.

In figure 10D a protein patterning process directly on catheter 2 is observed, with PEG polyethylene glycol and PDMS polydimethylsiloxane.

In the cases discussed relating to figures 10A, 10B and 10C the micro/nanofilms, the differentiated iPSCs are subsequently made to adhere to the catheter 2, thanks to suitable known techniques, suitable for creating a stable interaction over time, resistant to stress and strain, and able not to negatively interfere with the contraction transmission from the cells on the micro/nanofilm to the catheter 2. Among the known potential usable techniques, it can be listed plasma and oxygen plasma treatments, which allow the generation of reactive surfaces to stabilize the adhesion between the microfilm and the catheter 2 itself. This technique can be coupled with the use of 3-Aminopropyltriethoxysilane (APTES), to create covalent bonds that are stable over time (see [29]), or with the use of polyvinyl alcohol (PVA), so as to favor the bonds between hydrophobic materials such as PDMS, and the PLLA (see [30]).

Alternatively, the micro/nanofilms can adhere into catheter 2 before the iPSC seeding and differentiation, which would then take place directly in the final catheter configuration.

If catheter 2 and micro/nanofilm are made with silicone material, the interaction between the two components can be stabilized by *curing* the silicone catheter 2 with the partially polymerized micro/nano film.

A further alternative concerns the creation of a pattern directly on the surface of the catheter 2 itself, thus avoiding the adhesion step of the micro/nanofilm to the catheter (figure 10B and 10D). This solution can be adopted by reconfiguring an expendable mold with a topography surface, capable of generating anisotropic channels on the catheter itself for physical patterning, or by creating a PDMS mold for protein patterning.

Each of the biohybrid actuators 31, 32, 33, and 34 must be able to exert a force and a stroke compatible with the mechanical features of the materials of the catheter 2 itself.

The contraction of the cells of the biohybrid actuators 31, 32, 33, and 34 must allow the tip, or the distal end 12 of the catheter 2, to be flexed at such an angle as to allow it to move even between the most tortuous vascular branches.

The control of the distal actuation at the tip is possible thanks to a genetic modification operated on the iPSCs, which makes the four biohybrid actuators 31, 32, 33, and 34 responsive to light stimuli with different wavelengths, as mentioned above.

The optical activation allows the system to be usable easily and safely, even for applications in deep tissues within the human body.

In fact, an activation of the muscles by electrical stimuli (as commonly happens, also in our body) would involve the need to integrate electrodes at the tip and to let electric currents flow inside the body. These would raise problems related to safety and possible unwanted cross-activations of different muscles integrated on the distal end of the catheter 2. Activation based on light radiation, on the other hand, avoids such problems.

As mentioned, suitable genetic modifications allow obtaining cells of the biohybrid actuators 31, 32, 33, and 34, which respond to light stimuli. For example, in embodiments, the expression of the Chronos or Chrimson proteins, respectively responsive to blue light (λ = 470 nm) and to far-red light (λ = 660 nm), is introduced (see [31]).

In the case of the exclusive culture of muscle cells, the latter can be genetically modified, to favor the expression of different photosensitive proteins on the four biohybrid actuators 31, 32, 33, and 34, making their contraction dependent on the light stimulus at different wavelengths.

This allows the operator to decide which of the four biohybrid actuators 31, 32, 33, and 34 to activate each time, by injecting polarized light with a specific wavelength into the optical fiber made in the longitudinal channel 21, thus obtaining the deformation of the distal end12 of catheter 2 in the desired direction.

In the case of the co-culture of muscle cells and neurons, it is the neurons that are genetically engineered. Following the optimization of the formation of neuromuscular junctions between neuronal and muscle cells, light enables the contraction of muscle cells to be activated by regulating (selectively, based on the wavelength) the depolarization of neuronal cells.

The light stimulus is controlled from the outside by the operator through a suitably controlled light source and is transmitted along the microcatheter being itself an optical fiber-based on polymeric material. This manufacturing process is possible starting from lithographic processes (see [32]).

Inside the walls of the medical device 1, and in particular of the catheter 2, there is, as mentioned, an appropriate coating, already described above, which allows a total internal reflection of the light for the transmission along its entire length. Alternatively, again as mentioned above, the optical radiation propagates along the cylindrical optical fiber 213, leaving the longitudinal channel 21.

Finally, the light stimulus can stimulate the biohybrid actuators 31, 32, 33, and 34 thanks to small holes or openings on the tip of the catheter, and also thanks to the transparency of the micro/nanofilms possibly integrated in the system (see [33]), which, as mentioned, form the actuation portion 212.

The operation of the medical device 1 described above is as follows.

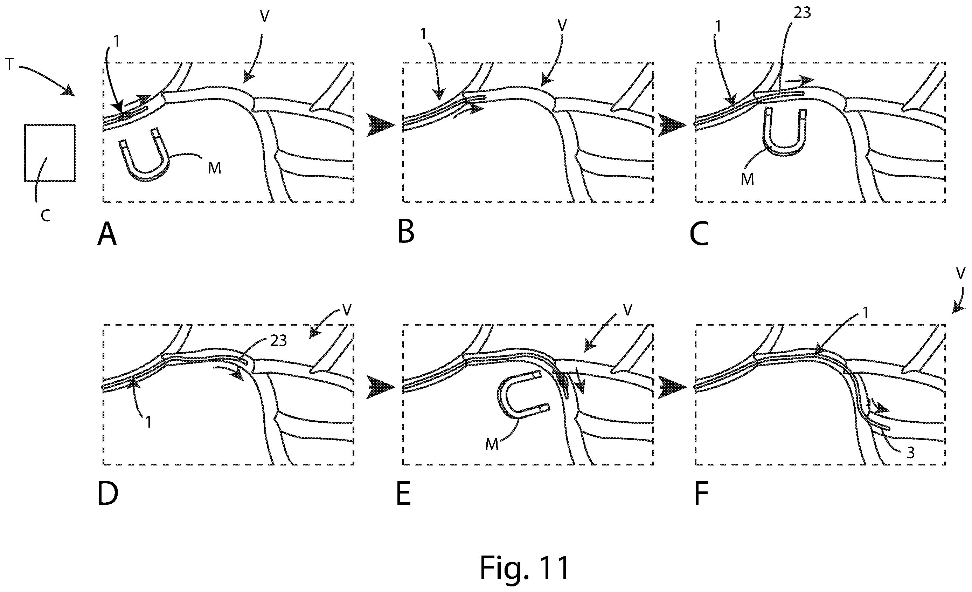

With reference to figure 11, various steps are observed, in which the medical device 1 is an example of the guiding of the catheter 2 thanks to magnetic and light stimuli, as well as the system for administering therapies T using the medical device 1.

In particular, the system for administering therapies T comprises, the medical device 1, a control unit C, and a navigation unit M.

More in detail, the distal end 23 of the catheter 2 is inserted into blood vessels V, i.e., in the patient's body, to which, for example, it is necessary to administer a pharmacological treatment in a specific area of the body, in which this treatment can be released in the distal end of said catheter 2. The control unit C is associated with the proximal end 22 of said catheter 2, to the introduction of actuation signals with suitable light radiation, to be transmitted through the longitudinal channel 21, up to the biohybrid actuators 31, 32, 33, and 34, to be able to move and selectively activate them.

Said navigation unit M is a magnetic system capable of interacting, by generating a magnetic field, with the particles responsive to magnetic fields incorporated in the material, which the catheter 2 is made of, so as to be able to drag it through the blood vessels V.

Figure 11 shows an example of guiding the catheter 2 inside a vascular system V. The catheter 2 is guided along the vessels V by means of the magnetic field generated by said navigation unit M (only schematically represented here by a magnet), and the luminous stimuli, realized by means of the light radiation emitted by the control unit C, to direct the bending of the distal end of the medical device 1. Therefore, there is a macroscopic control, by means of the navigation unit M, and a microscopic control, by means of the biohybrid actuators (31, 32, 33, 34).

In particular, in stet A, the navigation unit M moves the catheter 2 by means of a magnetic field along a blood vessel V.

In step B, by means of different electromagnetic radiations at different wavelengths generated by said control unit C, one of the biohybrid actuators 31, 32, 33, and 34 is activated, so as to orient the distal end 12, suitably equipped for example to deliver a drug.

In step C the medical device 1, and in particular the catheter 2, is again dragged by means of said navigation unit M, in step D the distal end 12 of the medical device 1 is reoriented by means of the control unit C, in step E the catheter 2 is now introduced into another capillary, and is dragged again by the navigation unit M, and finally, in step F, the distal end is once again oriented by inserting it into the optical fiber formed inside the longitudinal channel 21, suitably changing the wavelength of the activation signal generated by said control unit C.

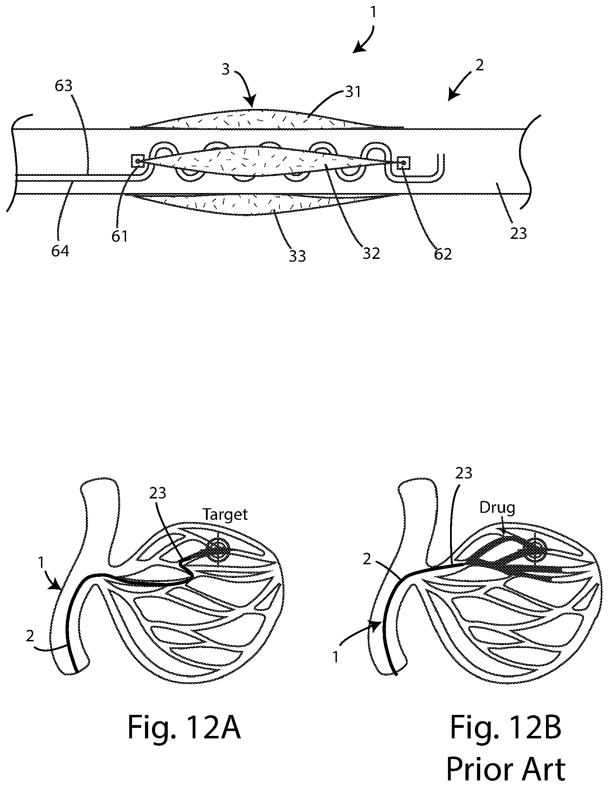

In this way, also referring to figure 12, it is possible to orient in tortuous and capillary areas of a body district, releasing (see figure 12A) the drug in the proximity of the target area, while with the catheters according to the prior art (see figure 12B) there is a non-specific distribution of the drug, due to the impossibility of reaching the target area with the distal end of catheter 2, forcing the operator to release the drug in a more distant area. Therefore, the catheters according to the prior art cannot arrive in a more specific way in the area to be treated.

With reference to figure 13, a second embodiment of the medical device 1 is observed. In particular, this embodiment of the medical device 1 differs from the previous one in that the catheter 2 does not have a waveguide inside and possibly it does not provide a longitudinal channel 21.

Furthermore, as better explained below, the activation mode of the biohybrid actuators 31, 32, 33, and 34 is based on electrical and non-optical stimuli, or, in general, by means of electromagnetic radiation.

In this case, in correspondence with each of the biohybrid actuators 31, 32, 33, or 34, there is a pair of electrodes 61 and 62, powered by respective electric tracks 63 and 64, so that it is possible, by means of suitable electrical actuation signals, selectively activating a respective biohybrid actuator 31, 32, 33, and 34 (in the case of the figure, the biohybrid actuator 32 is observed).

The electrodes 61 and 62 and the electric tracks 63 and 64 can be integrated into the structure of the catheter 2, or arranged in the longitudinal channel 21, if the catheter 2 provides for it.

The control unit C of the system for the administration of therapies T will generate electrical actuation signals for the selective activation of each of the biohybrid actuators 31, 32, 33, and 34.

The operation of the medical device 1 according to the second embodiment described, in addition to the system for administering therapies T, which is integral to this second embodiment of the medical device 1, is similar to that of the medical device according to the first embodiment, with the difference that in this case the actuation signals emitted by the control unit C, as mentioned, are not electromagnetic radiations in the visible wavelength, which pass through the wave guide, but are electrical signals transmitted through the electric tracks 63 and 64, for the activation of the electrodes 61 and 62 of each of the biohybrid actuators 31, 32, 33, and 34.

### Advantages

An advantage of the solution described is the fact that the medical device provides an actuation principle of a biohybrid nature for the construction of bio- and immune-compatible actuators (as they are based on cells deriving from the patient himself), soft and miniaturizable, to be applied to biomedical devices, including catheters. The arrangement of the actuation of the catheter on the distal end of the medical device 1 allows a movement agility of the device in different environments and applications, such as for example the endovascular field, for the administration of pharmacological treatments, nanomedicines, cerebral or hepatic chemoembolizations, etc.. The same principle of distal bioactuation of medical instruments could find application in other applications and at different scales, for example in the field of cardiology, surgical operations in the abdominal region, etc..

A further advantage of the medical device according to the present invention is that of facilitating the navigation in tortuous and difficult to reach areas, thanks to a distal actuation on board, based on the presence of muscle cells differentiated from human iPSCs. The use of living cells to implement the actuation system has the advantages of allowing the actuator to be scaled to micrometric dimensions, impossible to reach by artificial actuators and making the actuation system self-powered, making the device compatible with the immune system of the patient (these cells being able to derive from the patient himself), constituting an "autologous" medical tool (i.e., consisting, at least in part, of the patient's own cells).

Another advantage of the present invention is the possibility of intravascularly applying these devices for chemoembolization near tumor sites to reduce blood flow to the tumor, such as for cerebral aneurysms, or in the liver in the presence of a hepatocarcinoma. With the current devices on the market, it is necessary to make a mapping of the vessels before the intervention, given the difficulty that can be encountered in navigation in the presence of acute angles in the path. This procedure is necessary to then choose preformed devices (see [34]), or customize the bending of the catheter according to the conformation of the vessels in the target site (see [35]). A study on the treatment of arterial aneurysms by embolization showed that 19% of preformed devices require a modification of the initial shape, while 29% require repositioning on the way, due to the backlash given by the catheter on the vessel wall due to the rigid tip (see [35]). The operator must, therefore, have different devices, depending on the intervention or proceed with different customization procedures. Probable complications due to the aforementioned problems, or to the choice of the incorrect device, are for example represented by the accidental embolization of non-injured areas, due to the inability to reach a specific target site. In neurosurgery, in certain areas such as the spine, this situation can be disabling, due to possible consequent ischemic damage. Furthermore, these devices require a guide catheter to approach the target site, within which they are made sliding, which necessarily has a larger size than the catheter and, in fact, limiting (e.g., 0.5 mm, see [34]). By means of the medical device 1 according to the invention, which provides for an actuation in the distal end, it is possible to consider broader spectrum applications, not having the limitations given by the use of preformed devices, and allowing effective navigation in the patient's vascular tree and ultimately, a more effective therapy with fewer side effects.

### Bibliography

[1] M. Wallington et al., "30-day mortality after systemic anticancer treatment for breast and lung cancer in England: a population-based, observational study.," Lancet. Oncol., vol. 17, no. 9, pp. 1203-16, Sep. 2016.
[2] C. De Angelis, "Side effects related to systemic cancer treatment: are we changing the Promethean experience with molecularly targeted therapies?," Curr. Oncol., vol. 15, no. 4, pp. 198-9, Aug. 2008.
[3] A. Schroeder et al., "Treating metastatic cancer with nanotechnology," Nat. Rev. Cancer, vol. 12, no. 1, pp. 39-50, Jan. 2012.
[4] S. Wilhelm et al., "Analysis of nanoparticle delivery to tumours," Nat. Rev. Mater., vol. 1, no. 5, p. 16014, May 2016.
[5] K. M. Tsoi et al., "Mechanism of hard-nanomaterial clearance by the liver," Nat. Mater., vol. 15, no. 11, pp. 1212-1221, Nov. 2016.
[6] S. Sharifi, S. Behzadi, S. Laurent, M. L. Forrest, P. Stroeve, and M. Mahmoudi, "Toxicity of nanomaterials.," Chem. Soc. Rev., vol. 41, no. 6, pp. 2323-43, Mar. 2012.
[7] A. Favaro, L. Cerri, S. Galvan, F. Rodriguez Y Baena, and E. De Momi, "Automatic optimized 3d path planner for steerable catheters with heuristic search and uncertainty tolerance," in ICRA 2018, 2018, pp. 9-16.
[8] B. Tesfamariam, "Local arterial wall drug delivery using balloon catheter system," J. Control. Release, vol. 238, pp. 149-156, Sep. 2016.
[9] K. Kandarpa, "Catheter-based Techniques for Endovascular Local Drug Delivery," J. Vasc. Interv. Radiol., vol. 11, no. 2, pp. 419-423, Feb. 2000.
[10] L. Muller, M. Saeed, M. W. Wilson, and S. W. Hetts, "Remote control catheter navigation: options for guidance under MRI.," J. Cardiovasc. Magn. Reson., vol. 14, no. 1, p. 33, Jun. 2012.
[11] S. Nazarian et al., "Feasibility of Real-Time Magnetic Resonance Imaging for Catheter Guidance in Electrophysiology Studies," Circulation, vol. 118, no. 3, pp. 223-229, Jul. 2008.
[12] A. Fenster, G. Parraga, and J. Bax, "Three-dimensional ultrasound scanning.," Interface Focus, vol. 1, no. 4, pp. 503-19, Aug. 2011.
[13] S. Jeon et al., "A Magnetically Controlled Soft Microrobot Steering a Guidewire in a Three-Dimensional Phantom Vascular Network.," Soft Robot., vol. 6, no. 1, pp. 54-68, Feb. 2019.
[14] C. Heunis, J. Sikorski, and S. Misra, "Flexible Instruments for Endovascular Interventions: Improved Magnetic Steering, Actuation, and Image-Guided Surgical Instruments," IEEE Robot. Autom. Mag., vol. 25, no. 3, pp. 71-82, Sep. 2018.
[15] J. Rahmer, C. Stehning, and B. Gleich, "Spatially selective remote magnetic actuation of identical helical micromachines," Sci. Robot., vol. 2, no. 3, p. eaal2845, Feb. 2017.
[16] V. N. T. Le, N. H. Nguyen, K. Alameh, R. Weerasooriya, and P. Pratten, "Accurate modeling and positioning of a magnetically controlled catheter tip," Med. Phys., vol. 43, no. 2, pp. 650-663, Jan. 2016.
[17] S. M. Jeon and G. H. Jang, "Precise Steering and Unclogging Motions of a Catheter With a Rotary Magnetic Drill Tip Actuated by a Magnetic Navigation System," IEEE Trans. Magn., vol. 48, no. 11, pp. 4062-4065, Nov. 2012.
[18] J. L. Pons, Emerging actuator technologies : a micromechatronic approach. John Wiley & Sons, 2005.
[19] K. Shimizu et al., "Assembly of skeletal muscle cells on a Si-MEMS device and their generative force measurement," Biomed. Microdevices, vol. 12, no. 2, pp. 247-252, Apr. 2010.
[20] Y. Morimoto, H. Onoe, and S. Takeuchi, "Biohybrid robot powered by an antagonistic pair of skeletal muscle tissues," Sci. Robot., vol. 3, no. 18, p. eaat4440, May 2018.
[21] A. K. Yetisen et al., "Glucose-Sensitive Hydrogel Optical Fibers Functionalized with Phenylboronic Acid," Adv. Mater., vol. 29, no. 15, p. 1606380, Apr. 2017.
[22] C. D. Schaper and A. Miahnahri, "Polyvinyl alcohol templates for low cost, high resolution, complex printing," J. Vac. Sci. Technol. B Microelectron. Nanom. Struct., vol. 22, no. 6, p. 3323, Dec. 2004.
[23] Y. C. Chen, W. L. Tseng, and C. H. Lin, "PDMS-based optical leaky waveguide coated with self-assemble Au-NPs for bio-analytical detections," Int. J. Autom. Smart Technol., vol. 2, no. 1, pp. 63-68, 2012.
[24] M. Choi, M. Humar, S. Kim, and S.-H. Yun, "Step-Index Optical Fiber Made of Biocompatible Hydrogels," Adv. Mater., vol. 27, no. 27, pp. 4081-4086, Jul. 2015.
[25] J. Lenzi et al., "Differentiation of control and ALS mutant human iPSCs into functional skeletal muscle cells, a tool for the study of neuromuscolar diseases," Stem Cell Res., vol. 17, no. 1, pp. 140-147, Jul. 2016.
[26] T. A. Dixon et al., "Bioinspired Three-Dimensional Human Neuromuscular Junction Development in Suspended Hydrogel Arrays," Tissue Eng. Part C Methods, vol. 24, no. 6, pp. 346-359, Jun. 2018.
[27] S. Jana, S. K. L. Levengood, and M. Zhang, "Anisotropic materials for skeletal-muscle-tissue engineering," Adv. Mater., vol. 28, no. 48, pp. 10588-10612, Dec. 2016.
[28] Y. Zhao, H. Zeng, J. Nam, and S. Agarwal, "Fabrication of skeletal muscle constructs by topographic activation of cell alignment," Biotechnol Bioeng, vol. 102, no. 2, pp. 624-631, Feb. 2009.
[29] M. Lee et al., "Combination of PDMS microfilters and micromixers based on flexible thermoplastic films for size sorting and mixing of microparticles," J. Appl. Polym. Sci., vol. 132, no. 25, p. n/a-n/a, Jul. 2015.
[30] T. Trantidou, Y. Elani, E. Parsons, and O. Ces, "Hydrophilic surface modification of PDMS for droplet microfluidics using a simple, quick, and robust method via PVA deposition," Microsystems Nanoeng., vol. 3, no. 1, p. 16091, Dec. 2017.
[31] N. C. Klapoetke et al., "Independent optical excitation of distinct neural populations.," Nat. Methods, vol. 11, no. 3, pp. 338-46, Mar. 2014.
[32] A. Ersen and M. Sahin, "A PDMS-based optical waveguide for transcutaneous powering of microelectrode arrays," in 2016 38th Annual International Conference of the IEEE Engineering in Medicine and Biology Society (EMBC), 2016, pp. 4475-4478.
[33] S.-J. J. S. Park et al., "Phototactic guidance of a tissue-engineered soft-robotic ray.," vol. 353, no. 6295, pp. 158-62, Jul. 2016.
[34] B. Scientific, "DirexionTM and Direxion HI-FLOTM Torqueable Microcatheters." [Online]. Available: http://www.bostonscientific.com/en-US/products/embolization/Direxion_and_Direxion_HI-FLO_Microcatheters.html. [Accessed: 09-Apr-2019].
[35] T. Ishibashi et al., "Tailor-made shaping of microcatheters using three-dimensional printed vessel models for endovascular coil embolization," Comput. Biol. Med., vol. 77, pp. 59-63, Oct. 2016.
[36] X. Hu, A. Chen, Y. Luo, C. Zhang, and E. Zhang, "Steerable catheters for minimally invasive surgery: a review and future directions," Comput. Assist. Surg., vol. 23, no. 1, pp. 21-41, Jan. 2018.
[37] V. Kallem and N. J. Cowan, "Image Guidance of Flexible Tip-Steerable Needles.," IEEE Trans. Robot., vol. 25, no. 1, pp. 191-196, Feb. 2009.
[38] K. B. Reed et al., "Robot-assisted needle steering," IEEE Robot. Autom. Mag., vol. 18, no. 4, pp. 35-46, Dec. 2011.
[39] A. Majewicz, T. R. Wedlick, K. B. Reed, and A. M. Okamura, "Evaluation of Robotic Needle Steering in ex vivo Tissue.," IEEE Int. Conf. Robot. Autom. ICRA [proceedings]. IEEE Int. Conf. Robot. Autom., vol. 2010, pp. 2068-2073, May 2010.
[40] S. Okazawa, R. Ebrahimi, J. Chuang, S. E. Salcudean, and R. Rohling, "Hand-Held Steerable Needle Device," IEEE/ASME Trans. Mechatronics, vol. 10, no. 3, pp. 285-296, 2005.
[41] D. B. Camarillo, C. R. Carlson, and J. K. Salisbury, "Configuration Tracking for Continuum Manipulators With Coupled Tendon Drive," IEEE Trans. Robot., vol. 25, no. 4, pp. 798-808, Aug. 2009.
[42] K. Kai Xu and N. Simaan, "Intrinsic Wrench Estimation and Its Performance Index for Multisegment Continuum Robots," IEEE Trans. Robot., vol. 26, no. 3, pp. 555-561, Jun. 2010.
[43] Y. Kim, G. A. Parada, S. Liu, and X. Zhao, "Ferromagnetic soft continuum robots," Sci. Robot., vol. 4, no. 33, p. eaax7329, Aug. 2019.
[44] N. B. Morgan, "Medical shape memory alloy applications-the market and its products," Mater. Sci. Eng. A, vol. 378, pp. 16-23, 2004.
[45] H. Takizawa, H. Tosaka, R. Ohta, and S. Kaneko, Development of a Microfine Active Bending Catheter Equipped with MIF Tactile Sensors. IEEE, 1999.
[46] J. Z. Gul, Y. J. Yang, K. Y. Su, and K. H. Choi, "Omni Directional Multimaterial Soft Cylindrical Actuator and Its Application as a Steerable Catheter," Soft Robot., vol. 4, no. 3, pp. 224-240, Sep. 2017.
[47] R. J. Webster, J. M. Romano, and N. J. Cowan, "Mechanics of Precurved-Tube Continuum Robots," IEEE Trans. Robot., vol. 25, no. 1, 2009.
[48] L. Ricotti et al., "Biohybrid actuators for robotics: a review of devices actuated by living cells," Sci. Robot., vol. 2, no. 12, Nov. 2017.
[49] L. Vannozzi et al., "Self-assembly of polydimethylsiloxane structures from 2D to 3D for bio-hybrid actuation," Bioinspir. Biomim., vol. 10, no. 5, p. 056001, Aug. 2015.
[50] J. Xi, J. J. Schmidt, and C. D. Montemagno, "Self-assembled microdevices driven by muscle," Nat. Mater. 2005 42, vol. 4, no. 2, p. 180, Jan. 2005.
[51] Z. Li et al., "Biohybrid valveless pump-bot powered by engineered skeletal muscle," Proc. Natl. Acad. Sci. U. S. A., vol. 116, no. 5, pp. 1543-1548, Jan. 2019.
[52] R. Raman, C. Cvetkovic, and R. Bashir, "A modular approach to the design, fabrication, and characterization of muscle-powered biological machines," Nat. Protoc., vol. 12, no. 3, p. 519, Feb. 2017.
[53] R. Raman et al., "Optogenetic skeletal muscle-powered adaptive biological machines," Proc. Natl. Acad. Sci., vol. 113, no. 13, pp. 3497-3502, Mar. 2016.
[54] A. W. Feinberg, A. Feigel, S. S. Shevkoplyas, S. Sheehy, G. M. Whitesides, and K. K. Parker, "Muscular Thin Films for Building Actuators and Powering Devices," Science (80-. )., vol. 317, no. 5843, pp. 1366-1370, Sep. 2007.
[55] H. Yawo, T. Asano, S. Sakai, and T. Ishizuka, "Optogenetic manipulation of neural and non-neural functions," Dev. Growth Differ., vol. 55, no. 4, pp. 474-490, May 2013.
[56] T. Asano, T. Ishizua, and H. Yawo, "Optically controlled contraction of photosensitive skeletal muscle cells," Biotechnol. Bioeng., vol. 109, no. 1, pp. 199-204, Jan. 2012.
[57] L. Rao, Y. Qian, A. Khodabukus, T. Ribar, and N. Bursac, "Engineering human pluripotent stem cells into a functional skeletal muscle tissue," Nat. Commun., vol. 9, no. 1, p. 126, 2018.
[58] V. K. Singh, M. Kalsan, N. Kumar, A. Saini, and R. Chandra, "Induced pluripotent stem cells: applications in regenerative medicine, disease modeling, and drug discovery.," Front. cell Dev. Biol., vol. 3, p. 2, 2015.

The present invention has been described for illustrative but not limitative purposes, according to its preferred embodiments, but it is to be understood that modifications and/or changes can be introduced by those skilled in the art without departing from the relevant scope as defined in the enclosed claims.

## Claims

1. Medical device (1) for releasing drugs, functionalized nanometric vectors, medical equipment and the like, comprising
a catheter (2), having one proximal end (22) and one distal end (23), which, in use, can be inserted into the blood vessels (V) or a body area of a patient, wherein said distal end (23) is configured for releasing said drugs, functionalized nanometric vectors, medical equipment and the like,
**characterized**
**in that** it comprises one or more bio-hybrid actuators (31, 32, 33, 34), arranged in correspondence of said distal end (23) of said catheter (2),
wherein said one or more bio-hybrid actuator (31, 32, 33, 34) can be actuated by means of actuation signals, for orienting said distal end of said catheter (2).

2. Medical device (1) according to the preceding claim, **characterized in that** it comprises four distally integrated bio-hybrid actuators (31, 32, 33, 34).

3. Medical device (1) according to any one of the preceding claims, **characterized in that** said one or more bio-hybrid actuators (31, 32, 33, 34) are based on cells differentiated starting from iPSC (Induced Pluripotent Stem Cell), preferably derived from the patient himself.

4. Medical device (1) according to any one of the preceding claims, **characterized**
**in that** said one or more bio-hybrid actuators (31, 32, 33, 34) are based on optogenetically modified cells,
**in that** said actuation signals are electromagnetic radiations, in particular optical control or actuation signals, for actuating said one or more bio-hybrid actuators, and
**in that** said catheter (2) has an actuation portion (212), arranged in correspondence of said at least one bio-hybrid actuator (31, 32, 33, 34), through which said control electromagnetic radiations pass for reaching said at least one bio-hybrid actuator (31, 32, 33, 34) for actuating it.

5. Medical device (1) according to the preceding claim, **characterized**
**in that** said catheter (2) has a longitudinal channel (21), and
**in that** said catheter (2) comprises inside said longitudinal channel (21) an optical fiber, for transmitting said actuation optical signals.

6. Medical device (1) according to the preceding claim, **characterized in that** said longitudinal channel (21) has an internal reflecting wall (211), for reflecting and transmitting along the catheter (2) said actuation signals.

7. Medical device (1) according to the preceding claim, **characterized in that** said internal reflecting wall (211) that carries out said optical fiber is coated with a layer composed of gold nanoparticles.

8. Medical device (1) according to any one of claims 6 or 7, **characterized in that** said internal reflecting wall (211) that carries out said optical fiber is coated with alginate treated with calcium chloride, for providing a multilayer coating for the propagation of control electromagnetic radiation in said longitudinal channel (21).

9. Medical device (1) according to claim 5, **characterized in that** said catheter (2) comprises an optical fiber (213) having a cylindrical shape, so as to identify said longitudinal channel (21).

10. Medical device (1) according to any one of claims 4-9, **characterized in that** said actuation portion (212) is an opening, for allowing the passage of said control electromagnetic radiations of said actuation signal, or it is a portion of said transparent catheter (2), so as to allow the transmission of said control electromagnetic radiations of said actuation signal for reaching said one or more bio-hybrid actuators (31, 32, 33, 34).

11. Medical device (1) according to any one of the preceding claims when dependent on claim 3, **characterized in that** said one or more bio-hybrid actuators (31, 32, 33, 34) are each made of different groups of cells, optogenetically modified so as to respond to light radiation at different wavelengths, so that each of said bio-hybrid actuator (31, 32, 33, 34) can be actuated independently when control electromagnetic radiations are transmitted through said longitudinal channel (21).

12. Medical device (1) according to any one of claims 1-3, **characterized**
**in that** each of said bio-hybrid actuators (31, 32, 33, 34) comprises a couple of electrodes (61, 62),
**in that** it comprises electric tracks (63, 64) associated with each bio-hybrid actuator (31, 32, 33, 34), so that a respective bio-hybrid actuator (31, 32, 33, 34) can be selectively actuated by means of electrical actuation signals.

13. Medical device (1) according to the preceding claim, **characterized in that** said electric tracks (63, 64) are integrated in said catheter (2) or inserted in said longitudinal channel (21).

14. Medical device (1) according to any one of the preceding claims, **characterized in that** said catheter (2) comprises particles responsive to magnetic fields incorporated in the material of which it is made, as micro/nanoparticles of iron oxide (Fe3O4), neodymium iron boron (NdFeB) or maghemite (γ-Fe2O3).

15. Medical device (1) according to any one of the preceding claims, **characterized in that** said catheter (2) is made of
biocompatible elastomeric materials, such as polydimethylsiloxane (PDMS) and/or
polyurethanes that can be polymerized by temperature, electromagnetic or condensation radiation, wherein said polyurethanes are modulated in stiffness/elasticity as a function of isocyanate and hydroxyl groups, and/or
it comprises synthetic immuno- and emo-compatible hydrogels that can be modulated in stiffness, such as polyethylene glycol (PEG), in which the stiffness, comprising diacrylate groups (PEGDA).

16. System for administering therapies (T) for releasing drugs, functionalized nanometric vectors, medical equipment and the like, comprising
a medical device (1) according to any one of claims 1-15, wherein said distal end (23) of said catheter (2) can be inserted into the blood vessels (V) or the body of a patient,
a control unit (C) connected to the proximal end (22) of said catheter (2), for introducing signals with appropriate light radiation, to be transmitted by means of said longitudinal channel (21) up to said bio-hybrid actuators (31, 32, 33, 34), for selectively activating them, and
a navigation unit (M) capable of interacting by means of generating a magnetic field with the particles incorporated in the material of which the catheter (2) is made, so as to be able to move said catheter (2).

## Patentansprüche

1. Medizinische Vorrichtung (1) zur Freisetzung von Arzneimitteln, funktionalisierten nanometrischen Vektoren, medizinischer Ausrüstung und dergleichen, umfassend
einen Katheter (2) mit einem proximalen Ende (22) und einem distalen Ende (23), der im Gebrauch in die Blutgefäße (V) oder einen Körperbereich eines Patienten eingeführt werden kann, wobei das distale Ende (23) zur Freisetzung der Arzneimittel, der funktionalisierten nanometrischen Vektoren, der medizinischen Ausrüstung und dergleichen ausgebildet ist,
**dadurch gekennzeichnet**
**dass** sie einen oder mehrere biohybride Aktuatoren (31, 32, 33, 34) umfasst, die im Bereich des distalen Endes (23) des Katheters (2) angeordnet sind,
wobei der eine oder die mehreren biohybriden Aktuatoren (31, 32, 33, 34) mittels Betätigungssignalen betätigbar sind, um das distale Ende des Katheters (2) auszurichten.

2. Medizinische Vorrichtung (1) nach dem vorhergehenden Anspruch, **dadurch gekennzeichnet, dass** sie vier distal integrierte biohybride Aktuatoren (31, 32, 33, 34) umfasst.

3. Medizinische Vorrichtung (1) nach einem der vorhergehenden Ansprüche, **dadurch gekennzeichnet, dass** der eine oder die mehreren biohybriden Aktuatoren (31, 32, 33, 34) auf Zellen basieren, die ausgehend von iPS-Zellen (induzierten pluripotenten Stammzellen) differenziert wurden, vorzugsweise solchen, die vom Patienten selbst stammen.

4. Medizinische Vorrichtung (1) nach einem der vorhergehenden Ansprüche, **dadurch gekennzeichnet dass** der eine oder die mehreren biohybriden Aktuatoren (31, 32, 33, 34) auf optogenetisch modifizierten Zellen basieren,
dass die Betätigungssignale elektromagnetische Strahlungen, insbesondere optische Steuer- oder Betätigungssignale, zur Betätigung des einen oder der mehreren biohybriden Aktuatoren sind, und
dass der Katheter (2) einen Betätigungsabschnitt (212) aufweist, der im Bereich des mindestens einen biohybriden Aktuators (31, 32, 33, 34) angeordnet ist und durch den die elektromagnetischen Steuerstrahlungen hindurchtreten, um den mindestens einen biohybriden Aktuator (31, 32, 33, 34) zu erreichen und ihn zu betätigen.

5. Medizinische Vorrichtung (1) nach dem vorhergehenden Anspruch, **dadurch gekennzeichnet, dass** der Katheter (2) einen Längskanal (21) aufweist und dass der Katheter (2) innerhalb des Längskanals (21) eine optische Faser zur Übertragung der optischen Betätigungssignale umfasst.

6. Medizinische Vorrichtung (1) nach dem vorhergehenden Anspruch, **dadurch gekennzeichnet, dass** der Längskanal (21) eine innere reflektierende Wand (211) zum Reflektieren und Übertragen der Betätigungssignale entlang des Katheters (2) aufweist.

7. Medizinische Vorrichtung (1) nach dem vorhergehenden Anspruch, **dadurch gekennzeichnet, dass** die innere reflektierende Wand (211), welche die optische Faser bildet, mit einer aus Goldnanopartikeln bestehenden Schicht beschichtet ist.

8. Medizinische Vorrichtung (1) nach einem der Ansprüche 6 oder 7, **dadurch gekennzeichnet, dass** die innere reflektierende Wand (211), welche die optische Faser bildet, mit Calciumchlorid behandeltem Alginat beschichtet ist, um eine Mehrschichtbeschichtung für die Ausbreitung der elektromagnetischen Steuerstrahlung in dem Längskanal (21) bereitzustellen.

9. Medizinische Vorrichtung (1) nach Anspruch 5, **dadurch gekennzeichnet, dass** der Katheter (2) eine optische Faser (213) mit zylindrischer Form umfasst, um den Längskanal (21) zu definieren.

10. Medizinische Vorrichtung (1) nach einem der Ansprüche 4-9, **dadurch gekennzeichnet, dass** der Betätigungsabschnitt (212) eine Öffnung ist, um den Durchtritt der elektromagnetischen Steuerstrahlungen des Betätigungssignals zu ermöglichen, oder ein Abschnitt des transparenten Katheters (2) ist, um die Übertragung der elektromagnetischen Steuerstrahlungen des Betätigungssignals zum Erreichen des einen oder der mehreren biohybriden Aktuatoren (31, 32, 33, 34) zu ermöglichen.

11. Medizinische Vorrichtung (1) nach einem der vorhergehenden Ansprüche, sofern von Anspruch 3 abhängig, **dadurch gekennzeichnet, dass** der eine oder die mehreren biohybriden Aktuatoren (31, 32, 33, 34) jeweils aus unterschiedlichen Gruppen von Zellen bestehen, die optogenetisch derart modifiziert sind, dass sie auf Lichtstrahlung unterschiedlicher Wellenlängen ansprechen, sodass jeder der biohybriden Aktuatoren (31, 32, 33, 34) unabhängig betätigt werden kann, wenn elektromagnetische Steuerstrahlungen durch den Längskanal (21) übertragen werden.

12. Medizinische Vorrichtung (1) nach einem der Ansprüche 1-3, **dadurch gekennzeichnet dass** jeder der biohybriden Aktuatoren (31, 32, 33, 34) ein Paar Elektroden (61, 62) umfasst,
dass sie elektrische Leiterbahnen (63, 64) umfasst, die jedem biohybriden Aktuator (31, 32, 33, 34) zugeordnet sind, sodass ein jeweiliger biohybrider Aktuator (31, 32, 33, 34) mittels elektrischer Betätigungssignale selektiv betätigt werden kann.

13. Medizinische Vorrichtung (1) nach dem vorhergehenden Anspruch, **dadurch gekennzeichnet, dass** die elektrischen Leiterbahnen (63, 64) in den Katheter (2) integriert oder in den Längskanal (21) eingeführt sind.

14. Medizinische Vorrichtung (1) nach einem der vorhergehenden Ansprüche, **dadurch gekennzeichnet, dass** der Katheter (2) auf Magnetfelder ansprechende Partikel umfasst, die in das Material, aus dem er hergestellt ist, eingebettet sind, wie Mikro-/Nanopartikel aus Eisenoxid (Fe3O4), Neodym-Eisen-Bor (NdFeB) oder Maghemit (*γ*-Fe2O3).

15. Medizinische Vorrichtung (1) nach einem der vorhergehenden Ansprüche, **dadurch gekennzeichnet, dass** der Katheter (2) hergestellt ist aus
biokompatiblen elastomeren Materialien, wie Polydimethylsiloxan (PDMS) und/oder Polyurethanen, die durch Temperatur, elektromagnetische Strahlung oder Kondensation polymerisiert werden können, wobei die Polyurethane hinsichtlich Steifigkeit/Elastizität in Abhängigkeit von Isocyanat- und Hydroxylgruppen modulierbar sind, und/oder
dass er synthetische immun- und hämokompatible Hydrogele umfasst, die hinsichtlich ihrer Steifigkeit modulierbar sind, wie Polyethylenglykol (PEG), wobei die Steifigkeit durch Diacrylatgruppen (PEGDA) modulierbar ist.

16. System zur Therapieverabreichung (T) zur Freisetzung von Arzneimitteln, funktionalisierten nanometrischen Vektoren, medizinischer Ausrüstung und dergleichen, umfassend
eine medizinische Vorrichtung (1) nach einem der Ansprüche 1-15, wobei das distale Ende (23) des Katheters (2) in die Blutgefäße (V) oder den Körper eines Patienten eingeführt werden kann,
eine Steuereinheit (C), die mit dem proximalen Ende (22) des Katheters (2) verbunden ist, um Signale mit geeigneter Lichtstrahlung einzuleiten, die mittels des Längskanals (21) bis zu den biohybriden Aktuatoren (31, 32, 33, 34) übertragen werden, um diese selektiv zu aktivieren, und
eine Navigationseinheit (M), die durch Erzeugen eines Magnetfelds mit den in das Material, aus dem der Katheter (2) hergestellt ist, eingebetteten Partikeln wechselwirken kann, sodass der Katheter (2) bewegt werden kann.

## Revendications

1. Dispositif médical (1) destiné à la libération de médicaments, de vecteurs nanométriques fonctionnalisés, de matériel médical et analogues, comprenant
un cathéter (2), ayant une extrémité proximale (22) et une extrémité distale (23), qui, lors de l'utilisation, peut être inséré dans les vaisseaux sanguins (V) ou dans une zone corporelle d'un patient, dans lequel ladite extrémité distale (23) est configurée pour libérer lesdits médicaments, vecteurs nanométriques fonctionnalisés, matériel médical et analogues,
**caractérisé en ce qu'**il comprend un ou plusieurs actionneurs biohybrides (31, 32, 33, 34), disposés au niveau de ladite extrémité distale (23) dudit cathéter (2),
dans lequel lesdits un ou plusieurs actionneurs biohybrides (31, 32, 33, 34) peuvent être actionnés au moyen de signaux d'actionnement, afin d'orienter ladite extrémité distale dudit cathéter (2).

2. Dispositif médical (1) selon la revendication précédente, **caractérisé en ce qu'**il comprend quatre actionneurs biohybrides (31, 32, 33, 34) intégrés distalement.

3. Dispositif médical (1) selon l'une quelconque des revendications précédentes, **caractérisé en ce que** lesdits un ou plusieurs actionneurs biohybrides (31, 32, 33, 34) sont basés sur des cellules différenciées à partir de cellules iPSC (induced Pluripotent Stem Cell, cellules souches pluripotentes induites), de préférence dérivées du patient lui-même.

4. Dispositif médical (1) selon l'une quelconque des revendications précédentes, **caractérisé en ce que** lesdits un ou plusieurs actionneurs biohybrides (31, 32, 33, 34) sont basés sur des cellules modifiées par optogénétique,
**en ce que** lesdits signaux d'actionnement sont des rayonnements électromagnétiques, en particulier des signaux optiques de commande ou d'actionnement, destinés à actionner lesdits un ou plusieurs actionneurs biohybrides, et
**en ce que** ledit cathéter (2) présente une portion d'actionnement (212), disposée au niveau dudit au moins un actionneur biohybride (31, 32, 33, 34), à travers laquelle passent lesdits rayonnements électromagnétiques de commande pour atteindre ledit au moins un actionneur biohybride (31, 32, 33, 34) afin de l'actionner.

5. Dispositif médical (1) selon la revendication précédente, **caractérisé en ce que** ledit cathéter (2) présente un canal longitudinal (21), et
**en ce que** ledit cathéter (2) comprend, à l'intérieur dudit canal longitudinal (21), une fibre optique destinée à transmettre lesdits signaux optiques d'actionnement.

6. Dispositif médical (1) selon la revendication précédente, **caractérisé en ce que** ledit canal longitudinal (21) présente une paroi interne réfléchissante (211), destinée à réfléchir et à transmettre le long du cathéter (2) lesdits signaux d'actionnement.

7. Dispositif médical (1) selon la revendication précédente, **caractérisé en ce que** ladite paroi interne réfléchissante (211), qui réalise ladite fibre optique, est revêtue d'une couche composée de nanoparticules d'or.

8. Dispositif médical (1) selon l'une quelconque des revendications 6 ou 7, **caractérisé en ce que** ladite paroi interne réfléchissante (211), qui réalise ladite fibre optique, est revêtue d'alginate traité avec du chlorure de calcium, afin de fournir un revêtement multicouche pour la propagation du rayonnement électromagnétique de commande dans ledit canal longitudinal (21).

9. Dispositif médical (1) selon la revendication 5, **caractérisé en ce que** ledit cathéter (2) comprend une fibre optique (213) ayant une forme cylindrique, de manière à identifier ledit canal longitudinal (21).

10. Dispositif médical (1) selon l'une quelconque des revendications 4 à 9, **caractérisé en ce que** ladite portion d'actionnement (212) est une ouverture destinée à permettre le passage desdits rayonnements électromagnétiques de commande dudit signal d'actionnement, ou est une portion dudit cathéter transparente (2), de manière à permettre la transmission desdits rayonnements électromagnétiques de commande dudit signal d'actionnement afin d'atteindre lesdits un ou plusieurs actionneurs biohybrides (31, 32, 33, 34).

11. Dispositif médical (1) selon l'une quelconque des revendications précédentes lorsqu'elle dépend de la revendication 3, **caractérisé en ce que** lesdits un ou plusieurs actionneurs biohybrides (31, 32, 33, 34) sont chacun constitués de différents groupes de cellules, modifiées par optogénétique de manière à répondre à un rayonnement lumineux à différentes longueurs d'onde, de sorte que chacun desdits actionneurs biohybrides (31, 32, 33, 34) puisse être actionné indépendamment lorsque des rayonnements électromagnétiques de commande sont transmis à travers ledit canal longitudinal (21).

12. Dispositif médical (1) selon l'une quelconque des revendications 1 à 3, **caractérisé en ce que** chacun desdits actionneurs biohybrides (31, 32, 33, 34) comprend une paire d'électrodes (61, 62),
**en ce qu'**il comprend des pistes électriques (63, 64) associées à chaque actionneur biohybride (31, 32, 33, 34), de sorte qu'un actionneur biohybride respectif (31, 32, 33, 34) puisse être actionné sélectivement au moyen de signaux électriques d'actionnement.

13. Dispositif médical (1) selon la revendication précédente, **caractérisé en ce que** lesdites pistes électriques (63, 64) sont intégrées dans ledit cathéter (2) ou insérées dans ledit canal longitudinal (21).

14. Dispositif médical (1) selon l'une quelconque des revendications précédentes, **caractérisé en ce que** ledit cathéter (2) comprend des particules sensibles aux champs magnétiques incorporées dans le matériau dont il est constitué, telles que des micro/nanoparticules d'oxyde de fer (Fe3O4), de néodyme-fer-bore (NdFeB) ou de maghémite (*γ*-Fe2O3).

15. Dispositif médical (1) selon l'une quelconque des revendications précédentes, **caractérisé en ce que** ledit cathéter (2) est constitué de
matériaux élastomères biocompatibles, tels que le polydiméthylsiloxane (PDMS) et/ou
des polyuréthanes pouvant être polymérisés par la température, par un rayonnement électromagnétique ou de condensation, dans lequel lesdits polyuréthanes sont modulés en rigidité/élasticité en fonction des groupes isocyanate et hydroxyle, et/ou il comprend des hydrogels synthétiques immunocompatibles et hémocompatibles pouvant être modulés en rigidité, tels que le polyéthylène glycol (PEG), dans lequel la rigidité comprend des groupes diacrylate (PEGDA).

16. Système d'administration de thérapies (T) destiné à la libération de médicaments,
de vecteurs nanométriques fonctionnalisés, de matériel médical et analogues, comprenant
un dispositif médical (1) selon l'une quelconque des revendications 1 à 15, dans lequel ladite extrémité distale (23) dudit cathéter (2) peut être insérée dans les vaisseaux sanguins (V) ou dans le corps d'un patient,
une unité de commande (C) reliée à l'extrémité proximale (22) dudit cathéter (2), destinée à introduire des signaux avec un rayonnement lumineux approprié, à transmettre au moyen dudit canal longitudinal (21) jusqu'auxdits actionneurs biohybrides (31, 32, 33, 34), afin de les activer sélectivement, et
une unité de navigation (M) apte à interagir, au moyen de la génération d'un champ magnétique, avec les particules incorporées dans le matériau dont le cathéter (2) est constitué, de manière à pouvoir déplacer ledit cathéter (2).
